# EUROPÄISCHE PATENTSCHRIFT

(11) **EP 2 621 382 B1**
(45) Veröffentlichungstag und Bekanntmachung des Hinweises auf die Patenterteilung: **23.08.2017**
(21) Anmeldenummer: 11763651.4
(22) Anmeldetag: 28.09.2011
(51) Int. Cl.: A61B 17/70, A61B 17/68, A61B 17/80, A61B 17/88, A61F 2/00, A61F 2/30, A61F 2/44

(54) **LAMINA-IMPLANTATSATZ**
LAMINA IMPLANT SET
JEU D'IMPLANTS POUR LAME VERTÉBRALE

(30) Priorität: 28.09.2010 EP 10011329
(43) Veröffentlichungstag der Anmeldung: 07.08.2013
(73) Patentinhaber: FACET-LINK Inc., Rockaway NJ 07866 (US)
(72) Erfinder: JENSEN, Harm-Iven, 24214 Noer (DE); LINK, Helmut, D., 22397 Hamburg (DE)
(74) Vertreter: Glawe, Delfs, Moll
(86) Internationale Anmeldenummer: PCT/EP2011/066886
(87) Internationale Veröffentlichungsnummer: WO 2012/041914

(56) Entgegenhaltungen:
- EP-A1- 0 761 175
- EP-A2- 0 307 241
- WO-A1-2006/104487
- CN-A- 101 584 601
- US-A1- 2004 193 159
- US-A1- 2008 215 096
- US-A1- 2010 161 056

## Beschreibung

Die Erfindung betrifft einen Implantat-Satz für die Lamina eines Wirbels umfassend mehrere Implantate, die je einen Grundkörper mit Anlageflächen an dem Wirbel und eine Befestigungseinrichtung umfassen.

Die menschliche Wirbelsäule umfasst eine Vielzahl von Wirbeln, die lasttragend übereinander angeordnet und gelenkig miteinander verbunden sind. Die Wirbel unterscheiden sich in ihrer Gestalt je nach ihrer Anordnung in der Wirbelsäule, weisen jedoch einige Gemeinsamkeiten auf. So weist jeder Wirbel einen massiven Wirbelkörper auf mit zwei seitlich nach hinten vorstehenden knöchernen Vorsprüngen (Pedikel), die in ihrem hinteren Teil über einen knöchernen Bogen verbunden sind. Im Verbindungsbereich ist der Knochenbogen als eine breite Platte (Lamina) ausgebildet, und weist in seiner Mitte einen nach hinten abragenden spinalen Vorsprung auf. Der spinale Vorsprung wie auch zwei weitere transverse Vorsprünge an den Seitenflächen der Pedikel bilden Anlenkpunkte für Muskeln und Bänder. In dem Bereich, wo die Pedikel in die breite Lamina übergehen, sind auf jeder Seite ein oberer und ein unterer Gelenkvorsprung angeordnet. Sie bilden jeweils Teil eines Facettengelenks (Zygapophysialgelenkes) mit einem benachbarten oberen beziehungsweise unteren Wirbel. Weiter ist ein Wirbel mit seinem benachbarten oberen beziehungsweise unteren Wirbel über jeweils eine Bandscheibe verbunden, die unten beziehungsweise oben auf den verhältnismäßig ebenen Deckflächen des Wirbelkörpers angeordnet sind. Der von der Rückseite des Wirbelkörpers und dem Wirbelbogen umgrenzte Raum bildet einen Hohlraum, in welchem parallel zur Wirbelsäule laufende Nervenstränge aufgenommen sind.

Aufgrund von Krankheit oder Verletzungen kann es zu vielfältigen Arten von Rückenschmerzen kommen. Diese können insbesondere von Defekten an der Bandscheibe, an den Facettengelenken und/oder durch Einklemmungen beziehungsweise Einschnürungen der in dem Hohlraum verlaufenden Nervenstränge hervorgerufen sein. Für den letzteren Fall ist es bekannt, auf die Nervenstränge ausgeübten Druck durch Auswölbungen der Bandscheibe oder knöcherne Zuwachsungen im Bereich des Hohlkanals durch Entfernen dieser Auswölbungen bzw. Zuwachsungen zu vermeiden. Hierzu wird durch die Rückseite des Wirbelbogens, in der Regel also durch die Lamina, ein Zugang zu dem Hohlraum geschaffen, und dort mittels geeigneter und an sich bekannter Instrumente die störenden Zuwachsungen entfernt. Damit wird der Druck von den Nervensträngen genommen, woraufhin sich der durch den Druck induzierte Schmerz entsprechend verringert. Bei dieser auch als Laminektomie beziehungsweise Dekompression bekannten Methode wird der in der Lamina geschaffene Zugang, also die dort vorhandene Öffnung, nach der Operation meist nicht verschlossen. Es hat sich gezeigt, dass hierdurch die mechanische Stabilität des Wirbels geschwächt ist.

Die Erfindung hat sich zur Aufgabe gesetzt, die Entstehung solcher Folgeprobleme zu verringern beziehungsweise zu vermeiden.

Die erfindungsgemäße Lösung liegt in den Merkmalen der unabhängigen Ansprüche. Vorteilhafte Weiterbildungen der Erfindung sind Gegenstand der abhängigen Ansprüche.

Ein Implantat-Satz umfasst mehrere Verstärkungsimplantate zum Einsetzen in die Lamina des Wirbels, die jeweils einen Grundkörper mit Anlageflächen an dem Wirbel und eine Befestigungseinrichtung umfassen, wobei der Grundkörper eine Vorderfläche, Rückfläche und mediale sowie laterale Seitenfläche aufweist, und die mediale und laterale Seitenflächen zur Anlage an Schnittflächen der Lamina ausgebildet sind, wobei erfindungsgemäß vorgesehen ist, dass die mediale Seitenfläche gegenüber der lateralen Seitenfläche nach hinten versetzt ist, und wobei an der Rückfläche ein nach hinten abragender Ausleger mit einer seitlichen Anlagefläche zur Anlage an einem spinalen Vorsprung angeordnet ist. Die Verstärkungsimplantate des Satzes unterscheiden sich hinsichtlich des Abstands zwischen medialer und lateraler Seitenfläche voneinander. Die Erfindung beruht auf dem Gedanken, eine Mehrzahl von blockartigen Verstärkungsimplantaten bereitzustellen, die sich im Hinblick auf ihre Dicke unterscheiden, um so auch Resektionsöffnungen unterschiedlicher Weite in der Lamina ausfüllen um sicher verschließen zu können. Mit der Befestigungseinrichtung wird erreicht, dass das Verstärkungsimplantat sicher in seiner Lage in der Lamina fixiert ist. Dank des Verstärkungsimplantats wird der durch die Resektionsöffnung unterbrochene Wirbelbogen wieder vollständig geschlossen. Damit ist nicht nur ein besserer Schutz für die in den Hohlkanal verlaufenden Nervenstränge erreicht, sondern auch die mechanische Stabilität des Wirbelbogens wird wiederhergestellt und erreicht spätestens nach dem Einwachsen des Implantats wieder die ursprünglichen Werte.

Die Erfindung macht sich hierbei zunutze, dass bei der in der Praxis besonders häufig ausgeführten unilateralen Resektion (hierunter wird der Zugang durch die Lamina nur an einer Seite, also entweder links oder rechts des spinalen Vorsprungs verstanden) mit dem spinalen Vorsprung eine ausreichend große und mechanisch stabile Befestigungsmöglichkeit zur Verfügung steht, welche für die Befestigung des erfindungsgemäßen Verstärkungsimplantats genutzt wird. Durch die beanspruchte Form mit den zueinander versetzten Seitenflächen, die an den bei der Resektion geschaffenen Schnittflächen der Lamina anliegen, wird eine formgünstige Integration des Verstärkungselements in dem Wirbelbogen erreicht, und zwar derart, dass das Verstärkungsimplantat weder in störender Weise in den Hohlraum für die Nervenstränge hineinragt noch nach außen hin wesentlich aufträgt. Mit anderen Worten, der Grundkörper des Verstärkungsimplantats bleibt im Wesentlichen innerhalb des Bereichs, der vor der Resektion von dem entsprechenden Teil der Lamina ausgefüllt wurde. Der Gefahr unerwünschter Irritationen der Nervenstränge innerhalb des Hohlkanals wie auch des umliegenden Gewebes ist damit in entscheidender Weise entgegengewirkt.

Ein weiterer Vorteil des erfindungsgemäßen Verstärkungsimplantats ist, dass nach Auswahl des richtigen Verstärkungsimplantats aus dem Satz nur eben dieses Teil einzusetzen ist, und dass keine weiteren Montage- oder Einstellarbeiten in der Tiefe der Operationsstelle erforderlich sind. Es genügt, das Implantat in der passenden Größe einzusetzen und mittels der Befestigungseinrichtung, im einfachsten Fall eine Knochenschraube, an der vorgesehenen Stelle zu fixieren. Diese Einfachheit der Implantation transformiert sich so in Sicherheit vor Fehlimplantation und damit direkt in verbesserte Erfolgsaussichten.

In den meisten Fällen sind die Seitenflächen parallel zueinander, jedoch können sie eine nach vorne hin leicht zulaufende Keilform bilden, wobei der Keilwinkel zwischen 0 und 20° beträgt. Vorzugsweise ist der Keilwinkel kleiner als 10°, weiter vorzugsweise kleiner als 5°.

Aufgrund der versetzten Anordnung der Seitenflächen zueinander, sind die Vorder- und Rückflächen schief im Hinblick zu den beiden Seitenflächen, und zwar vorzugsweise sind sie im Wesentlichen parallel oder maximal mit einer Abweichung von 20° dazu ausgerichtet.

Mit Vorteil sind die Seitenflächen nicht nur nach vorne und hinten zueinander versetzt, sondern auch nach oben und unten. Der Grundkörper weist somit zweckmäßigerweise eine rhombenförmige Gestalt in zwei verschiedenen Ebenen auf. Der Rombuswinkel (kleinere Innenwinkel) beträgt hierbei vorzugsweise zwischen 35 und 75° in der Vertikalen bezogen auf die Unterseite und 30 bis 60° in einer Ebene orthogonal zur Rückfläche.

Die weiter vorne angeordnete laterale Seitenfläche bildet mit der Vorderfläche vorzugsweise einen verrundeten Spitzenwinkel. Damit ist das Einführen des Grundkörpers in die von der Resektion geschaffene Öffnung erleichtert, wobei die Verrundung ein Verhaken bei unebener Gestalt der Schnittflächen an der Lamina verhindert und der spitze Winkel ein Einführen gegebenenfalls unter leichter elastischer Aufweitung (zur Erreichung eines sogenannten Press-Fits) bis hin zur vollen Dicke erleichtert.

Um ein zu weites Einschieben des Verstärkungsimplantats in den Hohlraum zu verhindern, ist an einer Seitenfläche vorzugsweise ein schulterförmiger Vorsprung ausgebildet, der als Anschlag fungiert. Damit ist gewährleistet, dass ein sicherer Sitz des Implantats an der vorgesehenen Stelle auch ohne detaillierte visuelle Kontrolle erreicht werden kann und insbesondere wird dem entgegengewirkt, dass durch zu weites Einschieben des Implantats Druck auf die in dem Hohlraum befindlichen Nervenstränge ausgeübt wird. Der Gefahr von Operationsfehlern wird damit entscheidend entgegengewirkt. Es hat sich bewährt, dass der schulterförmige Vorsprung in einem Abstand von der Vorfläche angeordnet ist, welche etwa dem 0,8 bis 2,2-fachen der Dicke des Grundkörpers entspricht. Bewährt hat sich ein linearer Zusammenhang mit Offset, so dass beginnend mit einer Dicke von 3 mm der Abstand 6 mm beträgt, und für jeweils 1 mm zusätzlicher Dicke der Abstand um 0,5 mm ansteigt.

An dem nach hinten abragenden Ausleger ist zweckmäßigerweise ein Befestigungsloch vorgesehen. Damit erfolgt eine Sicherung nicht nur durch die seitliche Anlagefläche und deren Kraftschluss an dem spinalen Vorsprung, sondern es kann durch Einführen eines entsprechenden Befestigungsmittels (bspw. einer Schraube) eine formschlüssige Befestigung an dem spinalen Vorsprung erreicht werden. Vorzugsweise ist dazu das Befestigungsloch zur polyaxialen Aufnahme einer Schraube ausgebildet. Hierunter wird verstanden, dass die Schraube mit ihrem Kopf eine sichere flächige Auflage im Bereich des Befestigungslochs nicht nur in einer exakt zentrischen Lage hat, sondern auch bei Winkelabweichungen von bis zu 15° in jeder Richtung. Damit kann auch bei unterschiedlicher Anatomie des Wirbels die Schraube stets in einer für die Befestigung günstigen Orientierung eingesetzt sein, vorzugsweise eine Translamina-Schraube. Mit ihr kann eine besonders sichere Halterung in dem unversehrten, auf der anderen Seite des spinalen Vorsprungs liegenden Teil der Lamina erreicht werden. Es kann aber auch eine Schraubbefestigung direkt an dem spinalen Vorsprung vorgesehen sein; dies empfiehlt sich in der Regel dann, wenn auch der gegenüberliegende Teil der Lamina einen Defekt aufweist. Mit Vorteil ist hierzu eine Schraubdübeleinrichtung vorgesehen. Sie ermöglicht eine sichere Befestigung auch bei dünnem spinalem Vorsprung und in all solchen Fällen, bei denen aufgrund der Kleinheit der hier verwendeten Befestigungsmittel eine ausreichend sichere Kraftübertragung durch das Schraubgewinde allein nicht gewährleistet wäre. Sie umfasst einen Dübel und eine Dübelschraube. Der Dübel ist vorzugsweise von hülsenartiger Gestalt mit mehreren an einem nahen Ende verbundenen Segmenten, die an ihrem fernen Ende frei sind und nach außen weisenden Halterhaken aufweisen. Sie sind so dimensioniert, dass sie beim Einschieben des Dübels in den spinalen Vorsprung auf der anderen Seite herausragen und dort den Öffnungsrand hintergreifen. Vorzugsweise sind die Halterhaken mehrstufig mit zum festen Ende abnehmender Höhe angeordnet, um bei unterschiedlich dicken spinalen Vorsprüngen einen sicheren Halt zu erreichen.

Die medialen und lateralen Seitenflächen sind vorzugsweise mit Spikes versehen. Bewährte Formen für die Spikes sind Kegelspitzen, Pyramiden, prismatische V-förmige Erhebungen in unterschiedlicher Ausdehnung und Höhe. Mit Vorteil sind diese asymmetrisch ausgeführt, und zwar in der Weise, dass sie nach vorne eine größere Schrägung aufweisen als in Gegenrichtung. Damit wird das Einschieben des Implantats erleichtert, und es ergibt sich ein Widerhakeneffekt gegenüber einem unerwünschten Auswandern nach hinten. Damit kann eine sichere primäre Fixation erreicht werden. Um zusätzlich die sekundäre Fixation zu steigern, sind vorzugsweise die medialen und lateralen Seitenflächen mit einer knochenwachstumsfördernden Beschichtung versehen. Hierbei kann es sich insbesondere um Hydroxylapatit oder andere osteoinduktive Substanzen handeln.

Vorzugsweise ist an der hinteren Kante der lateralen Seitenfläche eine zur Seite abragende Fixierzunge vorgesehen. Sie ist so ausgebildet, dass sie im implantierten Zustand auf einer Außenfläche des sogenannten Pars aufliegt. Um in Abhängigkeit von der individuellen Anatomie eine gute Auflage zu erreichen, ist der Winkel der Fixierzunge zur lateralen Seitenfläche vorzugsweise veränderlich. Dies kann in praktisch besonders günstiger Weise wie eine biegsame Ausbildung der Fixierzunge erreicht werden, vorzugsweise mit einer Materialverdünnung im Bereich des Übergangs zwischen Fixierzunge und Grundkörper. Die Fixierzunge kann ein Befestigungsloch aufweisen, das mit Vorteil mehrere definierte Aufnahmepositionen für ein zweites Befestigungselement, insbesondere eine Pars-Schraube, aufweist. Die definierten Aufnahmepositionen ermöglichen es, unterschiedliche Positionen für die Pars-Schraube in Relation zu der Fixierzunge vorzusehen, wobei in jeder Position die Pars-Schraube - anders als in einem Langloch - formschlüssig gehaltert ist. Wie auch bei der Befestigung für die Translamina-Schraube sind vorzugsweise die Aufnahmepositionen der Fixierzunge zur polyaxialen Aufnahme ausgebildet. Damit kann die Pars-Schraube nicht nur mit einem translatorischen, sondern auch mit zwei rotatorischen Freiheitsgraden im Verhältnis zur Fixierzunge angeordnet sein, was auch bei schwieriger Anatomie eine sichere Befestigungsmöglichkeit ergibt. Der Winkelbereich für die polyaxiale Aufnahme beträgt vorzugsweise wiederum etwa ± 15° in jeder Richtung.

Das Verstärkungsimplantat weist vorzugsweise an seiner Rückfläche eine Werkzeugaufnahmeeinrichtung auf. Diese ermöglicht eine sichere Aufnahme und Halterung des Verstärkungsimplantats an einem zur Implantation dienenden Werkzeug. Dem Operateur ist es damit erleichtert, das Verstärkungsimplantat sicher und genau an den vorgesehenen Implantationsort zu bringen und dort zu befestigen. Vorzugsweise weist die Werkzeugaufnahmeeinrichtung dazu eine Längsnut auf. Sie kann einteilig oder aus mehreren (auch runden) Ausnehmungen gebildet sein. Damit kann eine eindeutige Orientierung des Verstärkungsimplantats zu dem Werkzeug erreicht werden. Vorteilhafterweise ist im Grund der Werkzeugaufnahmeeinrichtung ein Zuggewinde ausgebildet. Damit ist es im Zusammenspiel mit einer Halteschraube an dem Werkzeug ermöglicht, das Verstärkungsimplantat an dem Werkzeug zu sichern, und es so nicht nur gegenüber einem Herunterfallen und Verlieren zu schützen, sondern auch zum Erhalt einer winkelrichtigen Orientierung.

Vorzugsweise ist dazu ein Haltewerkzeug vorgesehen, und zwar in der Weise, dass es einen Fuß mit einem zur Interaktion mit der Werkzeugaufnahmeeinrichtung ausgebildeten länglichen Greiffuß aufweist. Dieser umfasst einen zum komplementären Eingreifen in die Längsnut ausgebildeten vorstehenden Bereich am vorderen Ende. Vorzugsweise umfasst es eine Verbindung des Greiffußes mit einem Handgriff an einem langen Hohlschaft, durch den ein in das Zuggewinde greifendes Spannelement geführt ist. Damit kann vom Handgriff aus das Verstärkungsimplantat an dem Greiffuß festgespannt werden zur sicheren Implantation und bei Erreichen des Implantationsorts von diesem gelöst werden, ohne dazu in der Tiefe der Operationsstelle tätig werden zu müssen. Es hat sich in der Praxis bewährt, an dem hinteren Ende des Hohlschafts einen seitlich abragenden Vorsprung anzuordnen, der eine vordefinierte Orientierung zu dem länglichen Greiffuß aufweist. Der Vorsprung kann vorzugsweise ein Teil des Griffs sein. Damit ist dem Operateur bereits mit dem Ergreifen des Instruments klar, in welcher Orientierung sich das winkelrichtig an dem Greiffuß aufgespannte Verstärkungsimplantat befindet. Die Gefahr von Fehlimplantation durch Fehlausrichtung ist dadurch entschärft.

Als Material für das Verstärkungsimplantat ist vorzugsweise eine Titanlegierung oder Reintitan vorgesehen. Diese bildet den Vorteil einer hohen Biokompatibilität in Kombination mit guter mechanischer Verarbeitbarkeit und Belastbarkeit. Weiter haben sich als Materialien Legierungen aus Titan-Aluminium-Vanadium, Titan-Niob-Vanadium oder Kobalt-Chrom-Molybden sowie körperverträgliche Kunststoffe, wie Polyethetherketon (PEEK) oder Kombinationen dieser Materialien bewährt.

Die Grundkörper des erfindungsgemäßen Satzes weisen verschiedene Abstände zwischen medialer und lateraler Seitenfläche (dieser Abstand wird als Dicke bezeichnet) auf. Bewährt hat sich ein Bereich zwischen 3 und 15 mm, wobei sich eine Abstufung um jeweils 2 mm als praktisch ausreichend erwiesen hat. Für einen fein abgestuften Implantat-Satz kann auch eine Millimeterabstufung vorgesehen sein.

Der erfindungsgemäße Satz umfasst vorzugsweise zusätzlich spiegelverkehrte Implantate, die ebenfalls in verschiedenen Dicken vorgesehen sind. Damit kann eine der Anatomie angepasste Versorgung sowohl in dem linken wie auch in dem rechten Bereich der Lamina erfolgen.

Die Erfindung wird nachfolgend unter Bezugnahme auf die beigefügte Zeichnung erläutert, in der vorteilhafte Ausführungsbeispiele dargestellt sind. Es zeigen:
- Fig. 1: eine Ansicht von unten eines ersten Ausführungsbeispiels ein rechtsseitiges Verstärkungsimplantat;
- Fig. 2: eine Ansicht von schräg hinten des in Figur 1 dargestellten Ausführungsbeispiels;
- Fig. 3: einen Satz mit Verstärkungsimplantat in verschiedener Dicke;
- Fig. 4: eine Rückansicht mit eingesetzter Befestigungsschraube;
- Fig. 5: eine Ansicht von rechts mit eingesetzter Befestigungsschraube;
- Fig. 6: ein Instrument zur Implantation;
- Fig. 7 a-c: einen Wirbel mit einer Lamina-Resektion mit eingesetztem Implantat aus einer Rückansicht von unten; und
- Fig. 8: ein Befestigungsmittel gemäß einem zweiten Ausführungsbeispiel.

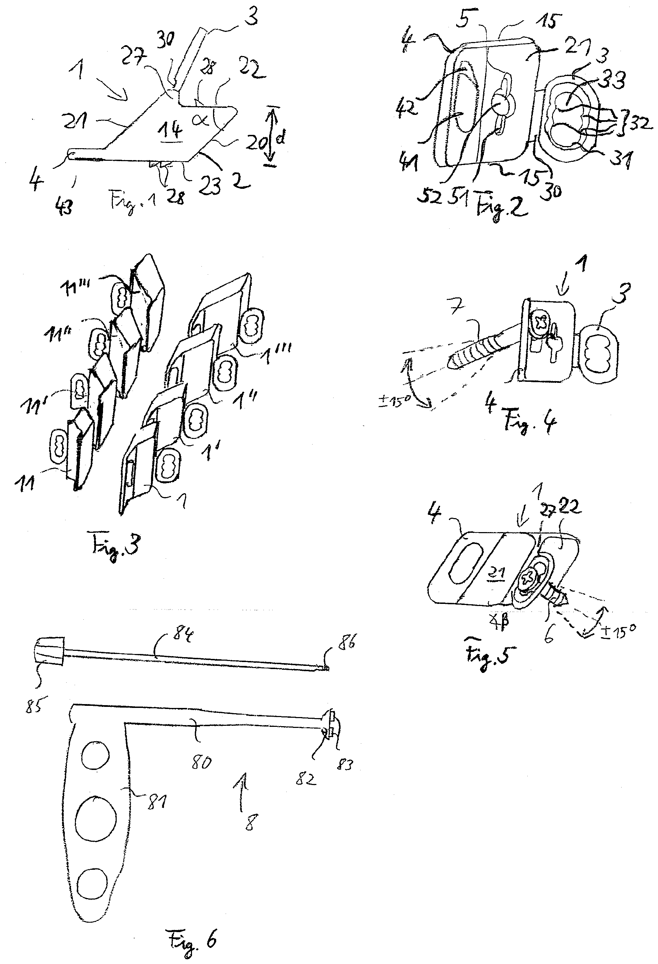

In Figur 1 ist ein erstes Ausführungsbeispiel für ein erfindungsgemäßes Verstärkungsimplantat dargestellt, welches in seiner Gesamtheit mit der Bezugsziffer 1 bezeichnet ist. Es weist eine nach vorne weisende schräge Vorderfläche 20, eine nach hinten weisende gleichfalls schräge Rückfläche 21 sowie eine laterale Seitenfläche 22 und eine mediale Seitenfläche 23 auf. Weiter ist eine Unterseite 14 sowie gegenüberliegend eine Oberseite 15 (s. Figur 2) vorgesehen, die über verrundete Kanten in die Vorderfläche 20 übergehen. An diesen Flächen umgrenzt ist ein Grundkörper 2, der eine doppelt rhombenförmige Gestalt aufweist.

Die Vorderfläche 20, Rückfläche 21 sowie die beiden Lateralflächen 22, 23 bilden einen Rombus mit einem Rombuswinkel α von 45° gemessen in einer Ebene orthogonal zur Rückfläche 21. (Unter Rombuswinkel wird der kleinere der Innenwinkel verstanden). Die lateralen und medialen Seitenflächen 22, 23 sind parallel zueinander ausgerichtet, es soll aber nicht ausgeschlossen sein, dass sie einen Keilwinkel bilden. Die Vorderfläche 20 und Rückfläche 21 sind ebenfalls parallel zueinander angeordnet. Weiter besteht eine rhombenförmige Gestalt in Bezug auf die Vorderfläche 20, Rückfläche 21, Unterseite 14 sowie Oberseite 15 (s. Figur 2). Hier beträgt der Rhombenwinkel β etwa 55°. An den Seitenflächen 22, 23 sind Spikes 28 angeordnet, die zur Primärfixation und nach vorne hin abgeschrägt sind.

Am hinteren Ende der medialen Seitenfläche ist im Bereich des Übergangs zur Rückfläche 21 ein nach hinten abragender Ausleger 4 angeordnet. Dieser weist auf seiner medialen Seite eine Anlagefläche 43 auf zur Anlage am spinalen Vorsprung eines Wirbels. Die Anlagefläche 43 und die mediale Seitenfläche 23 sind vorzugsweise in einer Ebene. Der Ausleger ist verhältnismäßig dünn und weist eine Materialstärke von bis zu 1,5 mm auf.

Im Bereich des Übergangs zwischen der lateralen Seitenfläche 22 und der Rückfläche 21 ist eine Anschlagschulter 27 ausgebildet. Die nach hinten weisende Seite ist schräg und bildet eine Ebene mit der Rückfläche 21, während ihre nach vorne weisende Seite lotrecht zur lateralen Seitenfläche 22 orientiert ist. Sie bildet damit mit ihrer Vorderseite einen Anschlag, welcher die Einschubtiefe des Implantats in die Resektionsöffnung begrenzt. Hierbei erfolgt der Einschub soweit, bis die Anschlagschulter 27 mit ihrer Vorderseite auf den Knochen der Lamina aufliegt.

An der Anschlagschulter 27 angelenkt ist eine Fixierzunge 3. Sie ist von im wesentlichen ovalartiger Gestalt und ist über einen Abschnitt mit einer Materialverdünnung 30 verbiegbar an der Anschlagschulter 27 des Grundkörpers 2 angeordnet. Die Fixierzunge 3 weist eine ähnliche Öffnung 31 auf, die auf ihren beiden Längsseiten über jeweils zwei Vorsprünge 32 in drei Bereiche unterteilt ist. Der Rand der Öffnung ist schräg geformt, so dass sich zusammen mit den Vorsprüngen 32 eine konische Auflagefläche für einen runden Aufnahmekopf ergibt, die an insgesamt drei Positionen in der Öffnung 1 gehaltert sein kann: eine obere Position, eine mittlere Position zwischen den Vorsprungspaaren 32 und einer unteren Position. Sie dienen zur Aufnahme einer Pars-Schraube (s. Figur 5). Dabei ist die Pars-Schraube 6 so in einer Aufnahmeposition 33 in der Öffnung 31 der Fixierzunge 3 gehaltert, dass sie in zwei Richtungen um ± 15° verschiedene Achsen (polyaxial) annehmen kann. Bedingt durch die verschiedenen Aufnahmepositionen 33 kann die Pars-Schraube 6 um 4 mm in der Öffnung 31 verschoben sein.

Eine ähnliche polyaxiale Aufnahme für eine Lamina-Schraube 7 ist in dem Ausleger 4 vorgesehen. Dazu ist eine Öffnung 41 ausgebildet, welche an ihrem Rand 42 ebenfalls Abschrägungen aufweist, um zusammen mit dem Schraubenkopf eine polyaxiale Lagerung um ± 15° (s. Figur 4) zu ermöglichen. Die Öffnung 41 ist als Langloch ausgebildet und ermöglicht es, die Lamina-Schraube über eine Länge von 3,5 mm in verschiedenen Positionen anzuordnen.

Weiter ist in der Rückfläche 21 eine Werkzeugaufnahmeeinrichtung 5 angeordnet. Sie umfasst eine Längsnut 51 mit einem in der Mitte ihres Nutgrunds angeordneten Sackloch mit einem Zuggewinde 52. Die Längsnut 51 dient zur winkeleindeutigen und rotationsgesicherten Aufnahme des Greiffußes eines Haltewerkzeugs, an das das Implantat über eine im Werkzeug enthaltene Spannschraube über das Zuggewinde 52 gezogen ist.

Eine Ausführungsform für ein entsprechendes Instrument 8 ist in Figur 6 dargestellt. Es umfasst einen lang gestreckten Schaft 80, der mit einer Hohlbohrung längs an der Mittelachse versehen ist. An einem Ende ist ein seitlich abragender Griff 81 winkelfest in Relation zu dem Hohlschaft 80 angeordnet. An dem anderen Ende des Hohlschafts 80 ist ein Greiffuß 82 ausgebildet, der an seinem äußeren Ende eine sich quer zur Achse des Hohlschafts 80 erstreckende Leiste aufweist. Sie ist so geformt, dass sie in Bezug auf ihre Länge und Breite zur komplementären Aufnahme in der Längsnut 51 in den Grundkörper 2 einführbar ist. In dem Hohlschaft 80 eingesetzt ist eine Spannschraube 84 mit einem Drehgriff 85 am griffseitigen Ende und einem Gewindekopf 86 am gegenüberliegenden Ende. Der Gewindekopf 86 ist so ausgebildet, dass er in das Gewinde 52 am Grund der Längsnut 51 fasst und dieses gegen den Greiffuß spannt. Damit ist das Implantat 1 verdreh- und verliersicher an dem Instrument 8 gehaltert. Durch entsprechende Orientierung des Griffstücks 81 weiß der Operateur genau, in welcher Winkellage sich das Implantat 1 befindet, und kann dieses zielgenau einführen, und zwar soweit bis die Anschlagschulter 27 ein weiteres Einschieben verhindert. Das Implantat 1 ist damit positioniert. Es brauchen dann nur noch mit geeigneten Bohrern und Schraubendrehern die zur weiteren Befestigung erforderlichen Pars-Schraube 6 und Lamina-Schraube 7 eingesetzt zu werden.

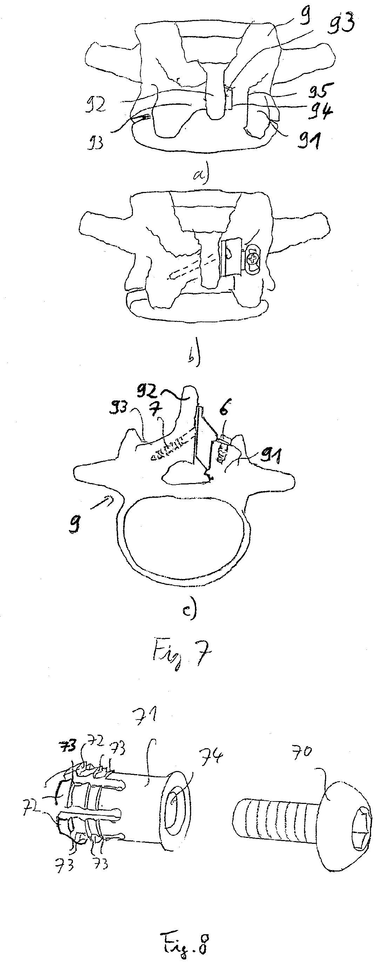

Am Beispiel für die Anordnung des Implantats in einem Wirbel 9 ist in Figur 7 a-c dargestellt. Figur 7a ist eine Rückansicht des Wirbels 9 mit einer Resektion im Bereich der Lamina 93 dargestellt, genauer gesagt im Bereich rechts des spinalen Vorsprungs 92. An dem linken und rechten Rand der Öffnung erkennt man so geschaffene Schnittflächen 94, 95. In diese Öffnung wird mittels des Instruments 8 wie vorstehend beschrieben das Implantat 1 eingesetzt. Es wird hinsichtlich seiner Dicke d so aus dem Satz (s. Fig. 3) ausgewählt, dass es den Zwischenraum zwischen den beiden Lamina-Schnittflächen 94, 95 vollständig ausfüllt. Damit greifen die Spikes 28 an den lateralen und medialen Seitenflächen 22, 23 des Grundkörpers 2 in die Schnittflächen 94, 95 ein und sorgen so für eine Primärbefestigung des Implantats. Um das Implantat an einem Auswandern und gegenüber einer Verdrehung weiter zu schützen, folgt eine weitere Verankerung durch die Pars-Schraube 6 im sogenannten Pars interarticularis 91 des Wirbels 9 und mittels der Lamina-Schraube 7 in dem auf der anderen Seite des spinalen Vorsprungs 92 gelegenen Teil der Lamina 93. Die Anordnung der Schraube ist zur Verdeutlichung in der teiltransparenten Darstellung in Figur 7c visualisiert.

Eine alternative Befestigung mit einer Schraubdübeleinrichtung ist in Figur 8 dargestellt. Sie umfasst einen Dübel 71, der mehrere durch Längsschlitze unterteilte Segmente 72 aufweist, die an einem Ende miteinander verbunden sind und deren anderes Ende frei ist. An dem freien Ende ist jeweils ein nach außen weisender Halterhaken 73 angeordnet, der zum Hintergreifen des Öffnungsrands der Bohrung ausgebildet ist, durch welchen der Dübel gesteckt ist. Um auch bei unterschiedlichen Längen von Durchgangsbohrungen ein hinreichend sicheres Hintergreifen zu erreichen, ist der Halterhaken vorzugsweise mehrstufig (im dargestellten Ausführungsbeispiel mit drei Stufen) ausgebildet. Jede Stufe weist eine geringere Höhe auf als die weiter außen liegende benachbarte. Der Dübel ist weiter mit einem Innengewinde 74 versehen. In dieses greift eine Fixierschraube 70, die mit einem entsprechenden Außengewinde versehen ist und welche anstelle der Lamina-Schraube 7 durch die Öffnung 41 in dem Ausleger 4 gesteckt ist. Damit kann eine sichere Verankerung an dem spinalen Vorsprung 92 erreicht werden.

Beispiele für Implantate 1 in verschiedenen Größen sind in Figur 3 dargestellt. Die Implantate 1, 1', 1" , 1"' sind im Wesentlichen gleich und unterscheiden sich nur in Bezug auf ihre Dicke d, d. h. in Abstand zwischen lateraler und medialer Seitenfläche 22 beziehungsweise 23. Weiter unterscheiden sie sich hinsichtlich des Abstands der Vorderseite der Schulter 27 von der verrundeten Vorderkante der lateralen Seitenfläche 22, welche bei dem dargestellten Ausführungsbeispiel etwa das 0,9-fache der Dicke d beträgt. Weiter umfasst der Satz gleichartige, jedoch spiegelverkehrte Implantate 11, 11', 11" , 11"', die zusammen einen Implantat-Satz 11 bilden. Damit können für Implantation linksseitig und rechtseitig in der Lamina jeweils eigene und an die Anatomie angepasste Implantate verwendet werden.

Die einzelnen Schritte der Implantation sind nachfolgend zusammengefasst. Nachdem eine Öffnung geschaffen wurde (vgl. Figur 7a) wird mit einem Probeimplantat die Weite der Öffnung und damit die geeignete Größe des Implantats 1 aus dem erfindungsgemäßen Implantat-Satz bestimmt. Das Probeimplantat enthält weiter Musterbohrungen am Ausleger 4 und an der Fixierzunge, um Bohrungen zur Aufnahme der Pars-Schraube 6 beziehungsweise Lamina-Schraube 7 mit dem Probeimplantat als Bohrlehre zu schaffen. In einem nächsten Schritt wird das Implantat auf das Werkzeug 8 in der gezeigten Weise aufgesetzt und gesichert und schließlich in den resezierten Raum in der Lamina 93 eingesetzt. Die Befestigungseinrichtungen zur Sicherung, insbesondere im Bereich des Auslegers 4 werden eingebracht. Durch Festdrehen der Befestigungsschrauben wird das Implantat endgültig gesichert.

## Patentansprüche

1. Implantat-Satz zum Einsetzen in die Lamina (93) eines Wirbels (9) umfassend mehrere Verstärkungsimplantate (1), die je einen Grundkörper (2) mit Anlageflächen (22, 23) an dem Wirbel (9) und eine Befestigungseinrichtung (7) umfassen, wobei der Grundkörper eine Vorderfläche (20), eine Rückfläche (21) und laterale und mediale Seitenflächen (22, 23) aufweist, wobei die Seitenflächen (22, 23) zur Anlage an Schnittflächen (94, 95) der Lamina (93) ausgebildet sind, **dadurch gekennzeichnet, dass** die mediale Seitenfläche (23) gegenüber der lateralen Seitenfläche (22) nach hinten versetzt ist, und an der Rückfläche (21) ein nach hinten abragender Ausleger (4) mit einer seitlichen Anlagefläche (43) zur Anlage an einem spinalen Vorsprung (92) des Wirbels (9) angeordnet ist.

2. Implantat-Satz nach Anspruch 1, **dadurch gekennzeichnet, dass** die Seitenflächen parallel bis leicht keilförmig zueinander orientiert sind, wobei ein Keilwinkel zwischen 0 und 20° beträgt.

3. Implantat-Satz nach Anspruch 1 oder 2, **dadurch gekennzeichnet, dass** die Vorderfläche (20) und die Rückfläche (21) schiefwinklig zu beiden Seitenflächen (22, 23) sind und vorzugsweise etwa parallel zueinander ausgerichtet sind.

4. Implantat-Satz nach Anspruch 2 oder 3, **dadurch gekennzeichnet, dass** der Grundkörper (2) eine rhombenförmige Gestalt in zwei Ebenen aufweist.

5. Implantat-Satz nach Anspruch 4, **dadurch gekennzeichnet, dass** ein Rombuswinkel α vorzugsweise zwischen 35 und 75° in einer Vertikalen und ein Rombuswinkel β zwischen 30 und 60° in einer Ebene orthogonal zur Rückfläche beträgt.

6. Implantat-Satz nach einem der vorangehenden Ansprüche, **dadurch gekennzeichnet, dass** die laterale Seitenfläche (22) mit der Vorderfläche (20) einen verrundeten Spitzenwinkel bildet.

7. Implantat-Satz nach einem der vorangehenden Ansprüche, **dadurch gekennzeichnet, dass** an einer Seitenfläche (22) ein schulterförmiger Vorsprung (27) ausgebildet ist, der als Anschlag fungiert.

8. Implantat-Satz nach Anspruch 7, **dadurch gekennzeichnet, dass** der Vorsprung (27) mit einem Abstand von der Kante zur Vorderfläche (20) angeordnet ist, der linear mit zunehmender Dicke wächst.

9. Implantat-Satz nach einem der vorangehenden Ansprüche, **dadurch gekennzeichnet, dass** an dem Ausleger (4) ein Befestigungsloch (41) vorgesehen ist.

10. Implantat-Satz nach Anspruch 9, **dadurch gekennzeichnet, dass** das Befestigungsloch (41) zur polyaxialen Aufnahme einer Schraube ausgebildet ist.

11. Implantat-Satz nach Anspruch 10, **dadurch gekennzeichnet, dass** die Befestigungseinrichtung eine Translamina-Schraube (7) umfasst.

12. Implantat-Satz nach Anspruch 10, **dadurch gekennzeichnet, dass** die Befestigungseinrichtung eine Schraubdübeleinrichtung (7') mit einem Dübel (71) und einer diesen greifenden Dübelschraube (70) ausgebildet ist.

13. Implantat-Satz nach Anspruch 12, **dadurch gekennzeichnet, dass** der Dübel (71) mehrere an einem Ende verbundene Segmente (72) aufweist, die an ihrem anderen Ende frei sind und nach außen wirkende Halterhaken (73) aufweisen.

14. Implantat-Satz nach Anspruch 13, **dadurch gekennzeichnet, dass** an den Segmenten (72) die Haltehaken mehrstufig mit zum festen Ende abnehmender Höhe angeordnet sind.

15. Implantat-Satz nach einem der vorangehenden Ansprüche, **dadurch gekennzeichnet, dass** die Seitenflächen (22, 23) mit Spikes (28) versehen sind, vorzugsweise nach vorne eine größere Schrägung aufweisen als nach hinten.

16. Implantat-Satz nach einem der vorangehenden Ansprüche, **dadurch gekennzeichnet, dass** die Seitenflächen (22, 23) mit einer knochenwachstumsfördernden Beschichtung versehen sind.

17. Implantat-Satz nach einem der vorangehenden Ansprüche, **dadurch gekennzeichnet, dass** an der Rückfläche (21) eine Werkzeugaufnahmeeinrichtung (5) angeordnet ist.

18. Implantat-Satz nach Anspruch 17, **dadurch gekennzeichnet, dass** die Werkzeugaufnahmeeinrichtung (5) eine Längsnut aufweist.

19. Implantat-Satz nach Anspruch 18, **dadurch gekennzeichnet, dass** am Grund der Längsnut ein Zuggewinde ausgebildet ist.

20. Implantat-Satz nach einem der Ansprüche 18 bis 19, **dadurch gekennzeichnet, dass** ein Haltewerkzeug vorgesehen ist, das einen zur Interaktion mit der Längsnut ausgebildeten Greiffuß aufweist.

21. Implantat-Satz nach Anspruch 20, **dadurch gekennzeichnet, dass** das Haltewerkzeug einen langen Hohlschaft (80) aufweist, durch den ein Spannelement (84) geführt ist, das in das Zuggewinde (52) greift.

22. Implantat-Satz nach Anspruch 20 oder 21, **dadurch gekennzeichnet, dass** am hinteren Ende des Hohlschafts (80) ein seitlich abragender Vorsprung (81) vorgesehen ist, der eine vordefinierte Orientierung zu dem länglichen Greiffuß (82) aufweist.

23. Implantat-Satz nach einem der vorangehenden Ansprüche, **dadurch gekennzeichnet, dass** als Material eine Titanlegierung oder Reintitan verwendet wird.

24. Implantat-Satz nach einem der vorangehenden Ansprüche, **dadurch gekennzeichnet, dass** die Grundkörper (2) verschiedene Dicken aufweisen im Bereich von 3 bis 15 mm.

25. Implantat-Satz nach einem der vorangehenden Ansprüche, **dadurch gekennzeichnet, dass** der Satz zusätzlich spiegelverkehrte Grundkörper (11) bis (11"') umfasst.

26. Implantat-Satz nach einem der vorangehenden Ansprüche, **dadurch gekennzeichnet, dass** an einer hinteren Kante der lateralen Seitenfläche (22) eine zur Seite abragende Fixierzunge (3) vorgesehen ist.

27. Implantat-Satz nach Anspruch 26, **dadurch gekennzeichnet, dass** die Fixierzunge (3) in ihrem Winkel zur lateralen Seitenfläche (22) veränderlich ist.

28. Implantat-Satz nach Anspruch 26 oder 27, **dadurch gekennzeichnet, dass** die Fixierzunge ein Befestigungsloch (31) aufweist, welches vorzugsweise mehrere definierte Aufnahmepositionen (33) für ein zweites Befestigungselement aufweist.

29. Implantat-Satz nach Anspruch 28, **dadurch gekennzeichnet, dass** die Aufnahmepositionen (33) zur polyaxialen Aufnahme einer Pars-Schraube (6) ausgebildet sind.

30. Implantat-Satz nach einem der Ansprüche 26 bis 29, **dadurch gekennzeichnet, dass** die zweite Befestigungseinrichtung eine Pars-Schraube (6) ist.

31. Implantat zum Einsetzen in die Lamina (93) eines Wirbels (9) mit einem Grundkörper (2) mit Anlageflächen (22, 23) an dem Wirbel und einer Befestigungseinrichtung, wobei der Grundkörper eine Vorderfläche (20), eine Rückfläche (21) und laterale und mediale Seitenflächen (22, 23) aufweist, wobei die Seitenflächen (22, 23) zu Anlage an Schnittflächen (94, 95) der Lamina (93) ausgebildet sind, **dadurch gekennzeichnet, dass** die mediale Seitenfläche (23) gegenüber der lateralen Seitenfläche (22) nach hinten versetzt ist, und an der Rückfläche (21) ein nach hinten abragender Ausleger (4) mit einer seitlichen Anlagefläche (43) zur Anlage an einem spinalen Vorsprung (92) des Wirbels (9) angeordnet ist.

32. Implantat nach Anspruch 31, **dadurch gekennzeichnet, dass** es nach einem der Ansprüche 2 bis 30 weitergebildet ist.

## Claims

1. Implant set for insertion into the lamina (93) of a vertebra (9), comprising several reinforcing implants (1), which each comprise a main body (2) with bearing surfaces (22, 23) on the vertebra (9) and a fastening device (7), wherein the main body has a front surface (20), a rear surface (21), and lateral and medial side surfaces (22, 23), wherein the side surfaces (22, 23) are designed to bear on sectioned surfaces (94, 95) of the lamina (93), **characterized in that** the medial side surface (23) is offset rearwardly in relation to the lateral side surface (22), and a rearwardly protruding extension (4) with a lateral bearing surface (43) to bear on a spinous process (92) of the vertebra (9) is arranged on the rear surface (21).

2. Implant set according to Claim 1, **characterized in that** the side surfaces are oriented parallel to each other or in a slightly wedge-shaped formation, **characterized in that** a wedge angle measures between 0 and 20°.

3. Implant set according to Claim 1 or 2, **characterized in that** the front surface (20) and the rear surface (21) are at an oblique angle to both side surfaces (22, 23) and are preferably oriented approximately parallel to each other.

4. Implant set according to Claim 2 or 3, **characterized in that** the main body (2) has a rhomboidal shape in two planes.

5. Implant set according to Claim 4, **characterized in that** a rhombus angle α preferably measures between 35 and 75° in a vertical, and a rhombus angle β measures between 30 and 60° in a plane orthogonal to the rear surface.

6. Implant set according to one of the preceding claims, **characterized in that** the lateral side surface (22) forms a rounded apex angle with the front surface (20).

7. Implant set according to one of the preceding claims, **characterized in that** a shoulder-shaped projection (27) is formed on one side surface (22) and functions as an abutment.

8. Implant set according to Claim 7, **characterized in that** the projection (27) is arranged at a distance from the edge to the front surface (20), which distance increases linearly with increasing thickness.

9. Implant set according to one of the preceding claims, **characterized in that** a fastening hole (41) is provided on the extension (4).

10. Implant set according to Claim 9, **characterized in that** the fastening hole (41) is designed for the polyaxial reception of a screw.

11. Implant set according to Claim 10, **characterized in that** the fastening device comprises a translaminar screw (7).

12. Implant set according to Claim 10, **characterized in that** the fastening device is designed as a screw dowel device (7') with a dowel (71) and with a dowel screw (70) engaging the latter.

13. Implant set according to Claim 12, **characterized in that** the dowel (71) has several segments (72), which are connected at one end and which, at their other end, are free and have outwardly acting retainer hooks (73).

14. Implant set according to Claim 13, **characterized in that** the retainer hooks on the segments (72) are arranged in several steps with a height increasing toward the fixed end.

15. Implant set according to one of the preceding claims, **characterized in that** the side surfaces (22, 23) are provided with spikes (28), preferably having a greater bevel toward the front than toward the rear.

16. Implant set according to one of the preceding claims, **characterized in that** the side surfaces (22, 23) are provided with a coating that promotes bone growth.

17. Implant set according to one of the preceding claims, **characterized in that** a tool receiver (5) is arranged on the rear surface (21).

18. Implant set according to Claim 17, **characterized in that** the tool receiver (5) has a longitudinal groove.

19. Implant set according to Claim 18, **characterized in that** a pulling thread is formed at the bottom of the longitudinal groove.

20. Implant set according to one of Claims 18 and 19, **characterized in that** a holding tool is provided, which has a gripper foot designed for interaction with the longitudinal groove.

21. Implant set according to Claim 20, **characterized in that** the holding tool has a long hollow shaft (80) through which a clamping element (84) is guided that engages in the pulling thread (52).

22. Implant set according to Claim 20 or 21, **characterized in that** a laterally extending projection (81) is provided at the rear end of the hollow shaft (80) and has a predefined orientation with respect to the elongate gripper foot (82).

23. Implant set according to one of the preceding claims, **characterized in that** the material used is a titanium alloy or pure titanium.

24. Implant set according to one of the preceding claims, **characterized in that** the main bodies (2) have different thicknesses in the range of 3 to 15 mm.

25. Implant set according to one of the preceding claims, **characterized in that** the set additionally comprises mirror-inverted main bodies (11) to (11'").

26. Implant set according to one of the preceding claims, **characterized in that** a laterally protruding fixing tongue (3) is provided on a rear edge of the lateral side surface (22).

27. Implant set according to Claim 26, **characterized in that** the fixing tongue (3) can be changed in terms of its angle to the lateral side surface (22).

28. Implant set according to Claim 26 or 27, **characterized in that** the fixing tongue has a fastening hole (31), which preferably has several defined receiving positions (33) for a second fastening element.

29. Implant set according to Claim 28, **characterized in that** the receiving positions (33) are designed for the polyaxial reception of a pars screw (6).

30. Implant set according to one of Claims 26 to 29, **characterized in that** the second fastening device is a pars screw (6).

31. Implant for insertion into the lamina (93) of a vertebra (9) on a main body (2) with bearing surfaces (22, 23) on the vertebra and a fastening device, wherein the main body has a front surface (20), a rear surface (21), and lateral and medial side surfaces (22, 23), wherein the side surfaces (22, 23) are designed to bear on sectioned surfaces (94, 95) of the lamina (93), **characterized in that** the medial side surface (23) is offset rearwardly in relation to the lateral side surface (22), and a rearwardly protruding extension (4) with a lateral bearing surface (43) to bear on a spinous process (92) of the vertebra (9) is arranged on the rear surface (21).

32. Implant according to Claim 31, **characterized in that** it is developed according to one of Claims 2 to 30.

## Revendications

1. Jeu d'implants pour l'insertion dans la lame vertébrale (93) d'une colonne vertébrale (9), comprenant plusieurs implants de renfort (1) qui comprennent chacun un corps de base (2) avec des faces d'appui (22, 23) contre la colonne vertébrale (9) et un dispositif de fixation (7), le corps de base présentant une face avant (20), une face arrière (21) et des faces latérales latérale et médiale (22, 23), les faces latérales (22, 23) étant réalisées de manière à venir en appui contre des faces de coupe (94, 95) de la lame vertébrale (93), **caractérisé en ce que** la face latérale médiale (23) est décalée vers l'arrière par rapport à la face latérale latérale (22), et une projection (4) faisant saillie vers l'arrière, avec une face d'appui latérale (43) prévue pour s'appliquer contre une saillie spinale (92) de la colonne vertébrale (9) étant disposée au niveau de la face arrière (21).

2. Jeu d'implants selon la revendication 1, **caractérisé en ce que** les faces latérales sont orientées parallèlement à légèrement en forme de coin l'une par rapport à l'autre, un angle de coin étant compris entre 0 et 20°.

3. Jeu d'implants selon la revendication 1 ou 2, **caractérisé en ce que** la face avant (20) et la face arrière (21) ont un angle oblique par rapport aux deux faces latérales (22, 23) et sont de préférence orientées approximativement parallèlement l'une à l'autre.

4. Jeu d'implants selon la revendication 2 ou 3, **caractérisé en ce que** le corps de base (2) présente une forme rhomboïde dans deux plans.

5. Jeu d'implants selon la revendication 4, **caractérisé en ce qu'**un angle du rhombe α est de préférence compris entre 35 et 75° par rapport à la verticale et un angle du rhombe β est compris entre 30 et 60° dans un plan perpendiculaire à la face arrière.

6. Jeu d'implants selon l'une quelconque des revendications précédentes, **caractérisé en ce que** la face latérale latérale (22) forme avec la face avant (20) un angle aigu arrondi.

7. Jeu d'implants selon l'une quelconque des revendications précédentes, **caractérisé en ce qu'**une saillie en forme d'épaulement (27) est réalisée au niveau d'une face latérale (22), laquelle sert de butée.

8. Jeu d'implants selon la revendication 7, **caractérisé en ce que** la saillie (27) est disposée à une distance de l'arête de la face avant (20) qui augmente de manière linéaire avec l'augmentation de l'épaisseur.

9. Jeu d'implants selon l'une quelconque des revendications précédentes, caractérisé en en ce qu'un trou de fixation (41) est prévu sur la projection (4).

10. Jeu d'implants selon la revendication 9, **caractérisé en ce que** le trou de fixation (41) est réalisé de manière à recevoir une vis de manière polyaxiale.

11. Jeu d'implants selon la revendication 10, **caractérisé en ce que** le dispositif de fixation comprend une vis translaminaire (7).

12. Jeu d'implants selon la revendication 10, **caractérisé en ce que** le dispositif de fixation est réalisé sous forme de dispositif à cheville filetée (7') avec une cheville (71) et une vis de cheville (70) s'engageant dans celle-ci.

13. Jeu d'implants selon la revendication 12, **caractérisé en ce que** la cheville (71) présente plusieurs segments (72) connectés au niveau d'une extrémité, qui sont libres à l'autre extrémité et qui présentent des crochets de fixation (73) agissant vers l'extérieur.

14. Jeu d'implants selon la revendication 13, **caractérisé en ce que** les crochets sont disposés au niveau des segments (72) en plusieurs étages avec une hauteur diminuant vers l'extrémité fixe.

15. Jeu d'implants selon l'une quelconque des revendications précédentes, **caractérisé en ce que** les faces latérales (22, 23) sont pourvues de pointes (28), de préférence présentent une plus grande inclinaison vers l'avant que vers l'arrière.

16. Jeu d'implants selon l'une quelconque des revendications précédentes, **caractérisé en ce que** les faces latérales (22, 23) sont pourvues d'un revêtement favorisant la croissance osseuse.

17. Jeu d'implants selon l'une quelconque des revendications précédentes, **caractérisé en ce qu'**un dispositif de réception d'outil (5) est disposé sur la face arrière (21).

18. Jeu d'implants selon la revendication 17, **caractérisé en ce que** le dispositif de réception d'outil (5) présente une rainure longitudinale.

19. Jeu d'implants selon la revendication 18, **caractérisé en ce qu'**un filetage de boulon de traction est réalisé à la base de rainure longitudinale.

20. Jeu d'implants selon l'une quelconque des revendications 18 à 19, **caractérisé en ce qu'**un outil de fixation est prévu, lequel présente un pied de préhension réalisé pour coopérer avec la rainure longitudinale.

21. Jeu d'implants selon la revendication 20, **caractérisé en ce que** l'outil de fixation présente une tige creuse longue (80) à travers laquelle est guidée un élément de serrage (84) qui s'engage dans le filetage de boulon de traction (52).

22. Jeu d'implants selon la revendication 20 ou 21, **caractérisé en ce qu'**une saillie (81) faisant saillie latéralement est prévue au niveau de l'extrémité arrière de la tige creuse (80), laquelle présente une orientation prédéfinie par rapport au pied de préhension oblong (82).

23. Jeu d'implants selon l'une quelconque des revendications précédentes, **caractérisé en ce que** l'on utilise en tant que matériau un alliage de titane ou du titane pur.

24. Jeu d'implants selon l'une quelconque des revendications précédentes, **caractérisé en ce que** les corps de base (2) présentent des épaisseurs différentes dans une plage de 3 à 15 mm.

25. Jeu d'implants selon l'une quelconque des revendications précédentes, **caractérisé en ce que** le jeu comprend en outre des corps de base à symétrie inversée (11) à (11"').

26. Jeu d'implants selon l'une quelconque des revendications précédentes, **caractérisé en ce qu'**une langue de fixation (3) faisant saillie latéralement est prévue au niveau d'une arête arrière de la face latérale latérale (22).

27. Jeu d'implants selon la revendication 26, **caractérisé en ce que** l'angle de la langue de fixation (3) peut être varié par rapport à la face latérale latérale (22).

28. Jeu d'implants selon la revendication 26 ou 27, **caractérisé en ce que** la langue de fixation présente un trou de fixation (31) qui présente de préférence plusieurs positions de réception définies (33) pour un deuxième élément de fixation.

29. Jeu d'implants selon la revendication 28, **caractérisé en ce que** les positions de réception (33) sont réalisées en vue de la réception polyaxiale d'une vis de Pars (6).

30. Jeu d'implants selon l'une quelconque des revendications 26 à 29, **caractérisé en ce que** le deuxième dispositif de fixation est une vis de Pars (6).

31. Implant destiné à être inséré dans la lame vertébrale (93) d'une colonne vertébrale (9), comprenant un corps de base (2) avec des faces d'appui (22, 23) au niveau de la colonne vertébrale et un dispositif de fixation, le corps de base présentant une face avant (20), une face arrière (21) et des faces latérales latérale et médiale (22, 23), les faces latérales (22, 23) étant réalisées de manière à venir en appui contre des faces de coupe (94, 95) de la lame vertébrale (93), **caractérisé en ce que** la face latérale médiale (23) est décalée vers l'arrière par rapport à la face latérale latérale (22), et une projection (4) faisant saillie vers l'arrière, avec une face d'appui latérale (43) prévue pour s'appliquer contre une saillie spinale (92) de la colonne vertébrale (9) étant disposée au niveau de la face arrière (21).

32. Implant selon la revendication 31, **caractérisé en ce qu'**il est perfectionné selon l'une quelconque des revendications 2 à 30.
